# EUROPÄISCHE PATENTSCHRIFT

(11) **EP 0 315 215 B1**
(45) Veröffentlichungstag und Bekanntmachung des Hinweises auf die Patenterteilung: **03.02.1993**
(21) Anmeldenummer: 88118531.8
(22) Anmeldetag: 07.11.1988
(51) Int. Cl.: A61B 17/60

(54) **Vorrichtung zum externen Festlegen von Knochenfragmenten**
External fixation device for bone fragments
Dispositif de fixation externe pour fragments d'os

(30) Priorität: 05.11.1987 DE 3737617; 02.11.1988 DE 3837228
(43) Veröffentlichungstag der Anmeldung: 10.05.1989
(73) Patentinhaber: Sturtzkopf, Robert, D-81243 München (DE); Stedtfeld, Hans-Werner, Priv.Doz.Dr.med., D-90475 Nürnberg (DE)
(72) Erfinder: Sturtzkopf, Robert, D-81243 München (DE); Stedtfeld, Hans-Werner, Priv.Doz.Dr.med., D-90475 Nürnberg (DE)
(74) Vertreter: Hauck, Hans, Dipl.-Ing.

(56) Entgegenhaltungen:
- EP-A- 0 011 258
- WO-A-88/03395
- FR-A- 1 569 090
- US-A- 4 523 585

## Beschreibung

Die Erfindung betrifft eine Vorrichtung zum externen Festlegen von Knochenfragmenten.

Derartige als Fixateure bezeichnete Vorrichtungen sind bekannt (EP-A-0011258). Zur Befestigung des Fixateurs werden in jedes Knochenfragment in einem Abstand beidseitig der Bruchstelle Schrauben eingedreht, an denen externe Klemmbacken befestigt sind, die über je ein Kugelgelenk mit einer Führungssäule beziehungsweise einem Rohr verbunden sind, in dem die Führungssäule gleitend aufgenommen ist. Durch eine in eine Nut der Führungssäule greifende Schraube läßt sich der Fixateur in Längsrichtung feststellen sowie gegen Verdrehen sichern. Mittels des Fixateurs lassen sich die beiden Knochenfragmente gegenseitig ausrichten und in der ausgerichteten Lage unverrückbar festlegen, wobei alle auf die Knochenfragmente einwirkenden Kräfte über den Fixateur und nicht über den Bruchspalt übertragen werden.

In dem nun beginnenden Heilungsprozeß bildet sich in dem Bruchspalt zwischen den beiden Knochenfragmenten zunächst eine bindegewebsartige Substanz, bis dann der Bruchspalt endgültig von Knochensubstanz durchbaut wird. Für den Heilungsprozeß ist es förderlich, daß nach einer bestimmten Zeit der Fixateur gelöst wird und die Bruchstelle beim Aufbringen von Kräften gestaucht werden kann. Im unbelasteten Zustand der Bruchstelle besteht nämlich die Gefahr, daß die Bildung von Knochensubstanz im Bruchspalt verzögert wird und die Bruchstelle elastisch bleibt.

Die Annäherung der beiden Knochenfragmente nach Lösen des Fixateurs, also die Verengung des Bruchspaltes kann von einem Weggeber gemessen werden, der an den beiden teleskopartigen Körpern zu diesem Zweck befestigt wird (DE-A-3722595). Es handelt sich dabei um sehr geringe Wege, die in der Größenordnung von wenigen Millimetern und darunter liegen. Die gemessene Stauchung gibt einen Hinweis über den Verlauf des Heilungsprozesses.

Ein wesentlicher Nachteil bekannter Fixateure besteht darin, daß sich die Teleskopkörper verklemmen. Es wurde vorgeschlagen, die Reibeigenschaften zwischen den Teleskopkörpern durch eine Hartstoffbeschichtung zu verbessern (DE-A-3722595). Darüberhinaus weist aber auch eine mit verbesserten Gleiteigenschaften versehene Teleskopverbindung Nachteile auf. Ist der Fixateur gelöst, so ist die gegenseitige Führung der Körper nicht mehr unbedingt gewährleistet. Insbesondere können sich die Teleskopkörper gegenseitig verdrehen, wenn entsprechende Kräfte aufgebracht werden. Auch Biegekräfte treten in dem Fixateur auf, so daß eine zuverlässige Aussage über das Stauchen der Knochenfragmente nicht mehr getroffen werden kann.

Der Erfindung liegt demzufolge die Aufgabe zugrunde, die Vorrichtung der eingangs geschilderten Art so auszubilden, daß ein Verklemmen des Fixateurs nicht eintritt, die Verschiebung der Körper des Fixateurs stets proportional der aufgebrachten Last ist und der Fixateur eine verbesserte Führung erhält, die im gelösten Zustand unerwünschte Bewegungen der Knochenfragmente verhindert.

Die genannte Aufgabe ist erfindungsgemäß durch die Merkmale des Patentanspruchs 1 bzw. 17 gelöst.

Vorteilhafte Weiterbildungen der Erfindung ergeben sich aus den weiteren Ansprüchen.

Durch die Parallelogrammführung gemäß Patentanspruch 1 wird einerseits die Reibung zwischen den beiden Körpern des Fixateurs soweit herabgesetzt, daß die Einstellung des Fixateurs in Längsrichtung praktisch reibungsfrei erfolgt. Trotzdem sind die beiden Körper auch im gelösten Zustand über das Parallelogrammgelenk spielfrei geführt. Ein gegenseitiges Verdrehen ist nicht möglich.

In einer weiteren erfindungsgemäßen Lösung gemäß Patentanspruch 17 ist das Gelenk durch Federn ersetzt, die einen geringen axialen Federweg ermöglichen und in radialer Richtung im wesentlichen starr sind. Dabei wird die Bauweise der erfindungsgemäßen Vorrichtung besonders einfach und kompakt. Dies gilt insbesondere auch für die Ausführung, bei der die Feder von einer elastischen Masse gebildet wird.

Das Gelenk selbst ist so ausgebildet, daß es nur einen verhältnismäßig geringen Hub ausführt, der im Bereich von wenigen Millimetern und darunter liegt. Durch diese Hubbegrenzung ist es vermieden, daß die Längsachsen der beiden Hälften des Fixateurs in einem nennenswerten Maße sich seitlich verlagern, wenn sich das Gelenk bewegt. Dagegen wird die erforderliche Längsanpassung der beiden Hälften unabhängig vom Gelenk durch eine Längsverstellung in dem einen Körper vorgesehen, der zu diesem Zweck aus zwei Teilen besteht.

Der erfindungsgemäße Fixateur zeichnet sich ferner durch eine platzsparende Bauweise aus, ist sehr stabil und robust und läßt sich aus gängigen Materialien, insbesondere Aluminium herstellen.

Erfindungsgemäß ist ferner eine Verstelleinrichtung vorgesehen, mit der es möglich ist, eine Zugkraft bzw. eine Druckkraft auf die Knochenfragmente auszuüben, um den Abstand zwischen den beiden Knochenfragmenten zu verändern, nämlich die beiden Knochenfragmente über die vom Gelenk aufgebrachte Zugkraft zusammenzuziehen oder mittels der Druckkraft auseinanderzuschieben. Die Verstelleinrichtung verschwenkt die Laschen, so daß das Parallelogrammgelenk verlängert oder verkürzt wird.

In einer vorteilhaften Ausführungsform der Erfindung besteht die Verstelleinrichtung aus einer auf das Gelenk aufsetzbaren Klammer, die als doppelarmiger Hebel ausgebildet ist, um die Verschiebung des Gelenks herbeizuführen. Die Verstellbewegung wird über ein Laschenpaar des Gelenks eingeleitet. Das Gelenk kann in beiden Richtungen verschoben werden, je nachdem, von welcher Seite die Klammer auf das Laschenpaar wirkt. In einer besonders vorteilhaften Ausführungsform ist die Klammer umsetzbar, so daß einmal die Druckkraft erzeugt wird, während beim Umsetzen der Klammer durch Zusammenwirken mit dem anderen Laschenpaar die Zugkraft erzeugt wird.

Ferner ist es mittels einer einfachen Vorrichtung möglich, die auf die Knochenfragmente ausgeübte Kraft zu messen bzw. die erzeugte Kraft auf einen vorbestimmten Wert zu begrenzen.

Ferner ist es für den Heilungsprozeß von Vorteil, wenn die Längsbeweglichkeit der Vorrichtung in beiden Richtungen, also beim Längen und Stauchen gegen die Federkraft erfolgt. Erfindungsgemäß läßt sich diese Beweglichkeit mittels Blattfedern erzielen.

Schließlich ist erfindungsgemäß das bekannte Kugelgelenk für die Einstellung des Fixateurs gegenüber den an den Knochenfragmenten befestigten Klemmplatten durch eine Mehrfach-Drehgelenkverbindung ersetzt, mit der die Einstellung verbessert wird. Auch läßt sich die axiale Baulänge der Vorrichtung verkürzen.

Mehrere Ausführungsbeispiele der Erfindung sind nachstehend anhand der Zeichnung erläutert. Es zeigt:
- Figur 1: eine Seitenansicht eines Fixateurs,
- Figur 2: eine Ansicht der für das Parallelogrammgelenk erforderlichen Laschen,
- Figur 3: eine perspektivische Ansicht des Fixateurs nach Figur 1 und 2,
- Figur 4: eine perspektivische Ansicht der Klammer,
- Figur 5: einen Schnitt durch eine Druckschraube mit Kraftmessung,
- Figur 6: einen Schnitt durch ein Kugelgelenk,
- Figur 7: eine teilweise Darstellung des Scharniergelenks mit Blattfeder,
- Figur 8: eine Ausführungsform mit Blattfedern als Gelenk,
- Figur 9: eine vergrößerte Draufsicht auf eine Kreuzfeder,
- Figur 10: eine Ausführungsform mit einer elastischen Werkstoffmasse als Feder und
- Figur 11: ein teilweise Darstellung eines dreiachsigen Drehgelenks.

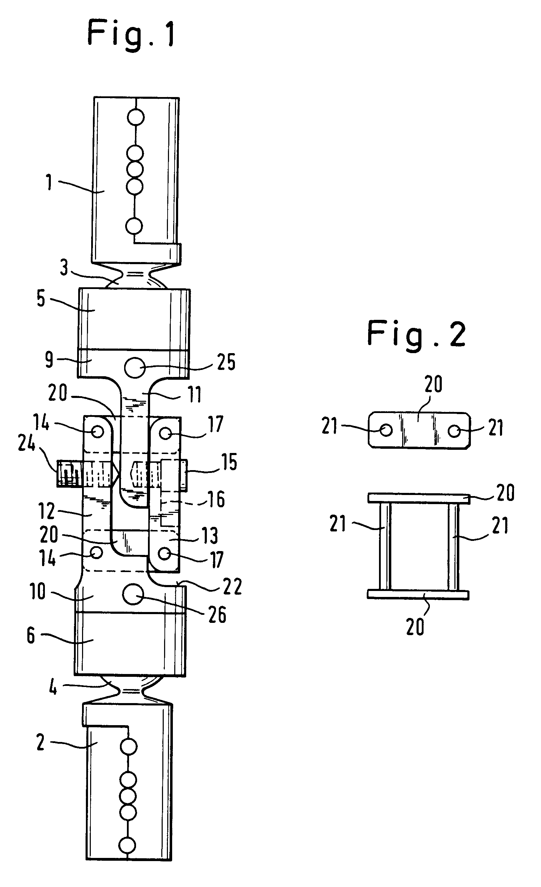

In Figur 1 weist der Fixateur jeweils obere und untere, geteilte Klemmbacken 1 und 2 auf, die an nicht dargestellten Schrauben befestigt werden, die in Knochenfragmente oberhalb und unterhalb eines Bruchspaltes eingeschraubt sind. An den Klemmbacken 1 und 2 ist jeweils eine Gelenkhälfte in Form einer Kugel 3 bzw. 4 angeformt, die in einer Gelenkhälfte 5 und 6 des Fixateuers drehbar und feststellbar gelagert ist. Mit diesen Gelenken läßt sich somit der Fixateur ausrichten und anpassen. Beide Klemmbacken 1, 2 sowie die Gelenkhälften 5, 6 sind zylindrisch.

An der Gelenkhälfte 5 ist lösbar ein Körper 9 befestigt, an dem eine etwa rechteckförmige, plattenförmige Verlängerung 11 angeformt ist. An dieser Verlängerung 11 ist ein Steg 13 befestigt, wobei eine Schraube 15 ein Langloch 16 in dem Steg 13 durchgreift und in eine Gewindebohrung der Verlängerung 11 einschraubbar ist. Somit läßt sich der Steg 13 in Längsrichtung gegenüber dem Körper 9 verstellen und festlegen.

An der anderen Gelenkhälfte 6 ist ebenfalls lösbar ein Körper 10 befestigt, der eine plattenförmige, rechteckförmige Verlängerung in Form eines Steges 12 trägt. Wie aus der Zeichnung ersichtlich ist, erstrecken sich die Stege 12 und 13 zueinander parallel und sind auf gleicher Höhe mit Bohrungen 14 bzw. 17 versehen, die sich in gleichen Abständen von der Längsachse des Fixateurs gegenüberliegen. Diese Bohrungen 14 und 17 bilden die Gelenkstellen des Parallelogrammgelenks. Die beiden Hälften werden durch die in Figur 2 gezeigten Laschen 20 miteinander verbunden. Die Laschen 20 sind durch Zapfen 21 aneinander befestigt, die in den Bohrungen 14, 17 drehbar gelagert sind.

Die Gelenkbewegung bzw. der Hub der beiden Körper 10 und 11 wird durch den Anschlag 22 begrenzt. Der Spalt zwischen dem Anschlag 22 am Körper 10 und der Stirnkante des Steges 13 beträgt nur wenige Millimeter.

Zum Festlegen des Fixateurs ist eine Druckschraube 24 vorgesehen, welche in dem Steg 12 eingeschraubt ist und mit ihrer Spitze gegen die Verlängerung 11 drückt.

Nach dem Befestigen der Klemmbacken 1, 2 an den in die Knochenfragmente eingesetzten Schrauben, wird die Schraube 15 angezogen und damit der Steg 13 im gewünschten axialen Abstand zum Körper 10 eingestellt. Dann wird das Parallelogrammgelenk durch Festziehen der Druckschraube 24 gesichert. Um Stauchkräfte auf den Bruchspalt aufzubringen wird die Druckschraube 24 gelöst. Dann können sich die beiden Körper 9 und 10 durch Bewegen des Parallelogrammgelenks gegenseitig annähern, wobei die Reibung vernachlässigt werden kann und die gegenseitige Führung der beiden Körper voll erhalten bleibt. In die zum Lösen der Gelenke dienenden Öffnungen 25 und 26, die insbesondere zum Einsetzen eines Imbusschlüssels ausgebildet sind, läßt sich bei Bedarf ein Wegmeßgeber in einfacher Weise einsetzen, so daß die gegenseitige Annäherung der beiden Körper einfach meßbar ist.

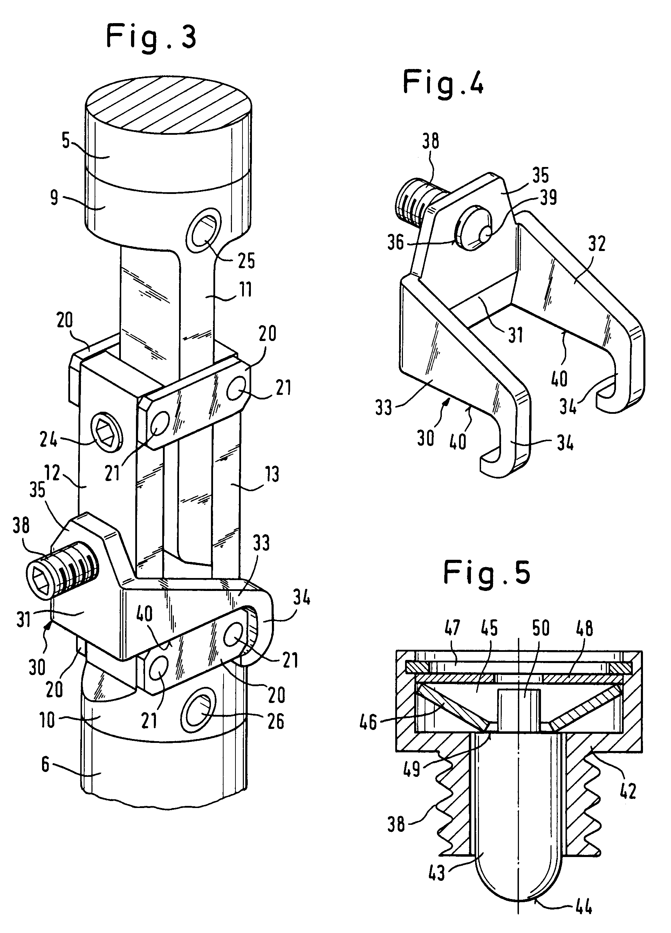

Eine Verstelleinrichtung in Form einer Klammer 30 besteht nach Figur 4 aus einem Steg 31, an dem zwei Arme 32, 33 angeformt sind, deren Enden 34 klauenförmig ausgebildet sind. Der Steg 31 weist eine Verlängerung 35 auf, in deren Gewindebohrung 36 eine Schraube 38 einsetzbar ist, die eine gerundete Spitze 39 aufweist.

Die Klammer 30 wird in der in Figur 3 dargestellten Weise an dem Gelenk angesetzt, so daß die klauenförmigen Enden 34 das untere Laschenpaar 20 umfassen. Mittels der Schraube 38, deren Ende 39 auf den Steg 12 drückt, läßt sich nun die Klammer verstellen, indem beim Einschrauben der Druckschraube 38 über die klauenförmigen Enden 34 die beiden Körper 9 und 10 auseinandergeschoben werden, wodurch eine Druckkraft auf die Knochenfragmente ausgeübt wird, so daß diese auseinandergeschoben werden.

Wird dagegen die Klammer 30 umgesetzt, so daß sie unterhalb dem oberen Laschenpaar 20 angesetzt wird, wobei die klauenförmigen Enden 34 nach oben weisen und das obere Laschenpaar 20 umschließen, so wird beim Einschrauben der Druckschraube 38 das Gelenk verkürzt und dabei eine Zugkraft erzeugt, welche die beiden Knochenfragmente zusammenzieht.

Wie erwähnt, wirkt die Klammer 30 als zweiarmiger Hebel. Der eine Hebelarm ist von dem Steg 31 mit der Verlängerung 35 gebildet, während der andere Hebelarm mit seinem Rand 40 jeweils an der Lasche 20 anliegt und mit dem klauenförmigen Ende 34 das Laschenende umgreift, so daß die Klammer nicht abgleitet.

In Figur 5 ist eine besondere Ausführungsform der Druckschraube 38 dargestellt, mit der die erzeugte Druck- bzw. Zugkraft begrenzbar ist und angezeigt wird. Hiernach ist in einer mit dem Gewinde zum Einschrauben in die Gewindebohrung der Klammer versehenen Buchse 42 ein Stift 43 geführt, dessen gerundete Spitze 44 auf den Steg 12 drückt. Die Buchse 42 weist am anderen Ende des Stiftes 43 einen Ringraum 45 zur Aufnahme einer Tellerfeder 46 auf, die einerseits an einer mit der Buchse 42 über einen Ring 47 verbundenen Abdeckscheibe 48 und andererseits an einer Schulter 49 des Stiftes 43 anliegt. Die Abdeckscheibe 48 weist eine mittlere Öffnung auf und der Stift 43 eine durch die Öffnung passende Verlängerung 50.

In Figur 5 ist die Tellerfeder 46 im ungespannten Zustand. Wird nun die Buchse 42 in die Klammer eingeschraubt und drückt der Stift 43 auf den Steg 12, so wird die beim Einschrauben ausgeübte Kraft über die Tellerfeder 46 auf den Stift 43 übertragen. Dabei verschiebt sich der Stift 43 gegenüber der Buchse 42 und verspannt die Tellerfeder 46. Tritt dabei die Verlängerung 50 in die Abdeckscheibe 48, so ist dies ein Maß für die erfolgte Verschiebung.

Durch Wahl entsprechender Tellerfedern läßt sich die vom Gelenk ausgeübte Druck- bzw. Zugkraft begrenzen. Tritt beim Anziehen der Schraube die Verlängerung 50 durch die Öffnung in der Abdeckscheibe 48, so daß die Verlängerung mit der Abdeckscheibe plan liegt, so ist die volle Federkraft der jeweiligen Tellerfeder 46 und damit eine bestimmte Druck- bzw. Zugkraft erreicht. Durch eine farbige Kennzeichnung können die Druckschrauben mit unterschiedlicher Tellerfeder voneinander unterschieden werden.

Sollen die Knochenfragmente zusammengeschoben oder auseinandergedrückt werden, so wird die Schraube 24 gelöst und die Klammer 30 an dem Gelenk so angesetzt, daß ein von der Feder 46 bestimmter Druck oder Zug auf die Knochenfragmente wirkt. In Verbindung mit dem reibungsfreien Parallelogramm wird so bei Zug der Heilungsprozeß durch die Kompression im Bruchspalt gefördert und kann bei Druck eine Verlängerung des gebrochenen Gliedes erzielt werden.

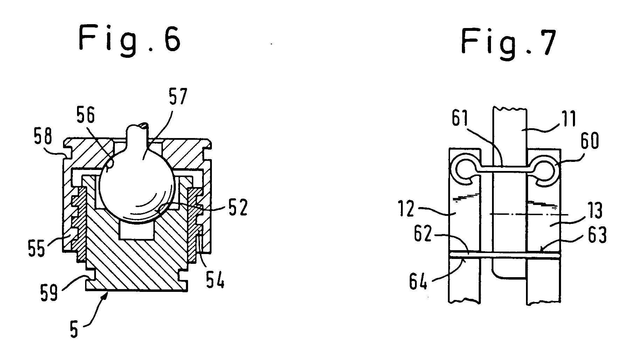

In Figur 6 ist die bereits erwähnte Gelenkhälfte 5 mit einer Pfanne 52 und mit einem Außengewinde 54 versehen. Besteht die Gelenkhälfte 5 aus Aluminium, so ist das Außengewinde 54 vorzugsweise an zwei Halbschalen aus Stahl angeordnet. Mittels einer Überwurfmutter 55 mit entsprechendem Gewinde und Pfanne 56 wird die Kugel 57 der anderen nicht dargestellten Gelenkhälfte, die in üblicher Weise mit Schrauben am Knochenfragment befestigt ist, auf den Sitz 52 gedrückt. Das Festziehen erfolgt durch einen die Überwurfmutter 55 bogenförmig umgreifenden Hakenschlüssel, der in die Aussparung 58 einsetzbar ist. Mit einem ähnlichen Hakenschlüssel, der in die Aussparungen 59 der Gelenkhälfte 5 greift, kann beim Anziehen der Überwurfmutter gegengehalten werden, so daß auf das Gelenk und damit auf die Knochenfragmente keine Kraft ausgeübt wird.

Ferner kann es für den Heilungsverlauf vorteilhaft sein, wenn auf die Bruchstelle ein pulsierender Druck bzw. Zug ausgeübt wird. Dies kann beispielsweise dann erfolgen, wenn der Patient ruht. Hierzu wird die Druckschraube 38 durch eine entsprechende Schraube mit angebautem hydraulischen Zylinder ersetzt, der an eine hydraulische Einrichtung zum Erzeugen eines pulsierenden Druckes, der auf den Stift 43 wirkt, angeschlossen wird. Es kann auch eine pneumatische oder elektrische Betätigung vorgesehen sein.

Es hat sich auch als vorteilhaft erwiesen, die beiden Körper 12 und 13 in einer Mittelstellung zu halten und eine Auslenkung in beiden Richtungen gegen Federkraft zuzulassen. Dies begünstigt die Knochenbildung im Bruchspalt in besonderer Weise. Wie aus Figur 7 hervorgeht, kann wenigstens ein Laschenpaar des Gelenks durch Blattfedern 60 ersetzt werden, deren Enden in entsprechende Ausnehmungen der Stege 12, 13 eingesetzt und darin befestigt werden, so daß das Längsverschieben des Gelenks aus der dargestellten Mittellage heraus durch entsprechendes Abbiegen des geraden Teils 61 der Blattfeder möglich ist.

Es kann auch eine Blattfeder 62 in einer Nut 63 im Steg 13 befestigt sein und greift das andere Ende der Blattfeder 62 lose in einen Schlitz 64 des anderen Steges 12.

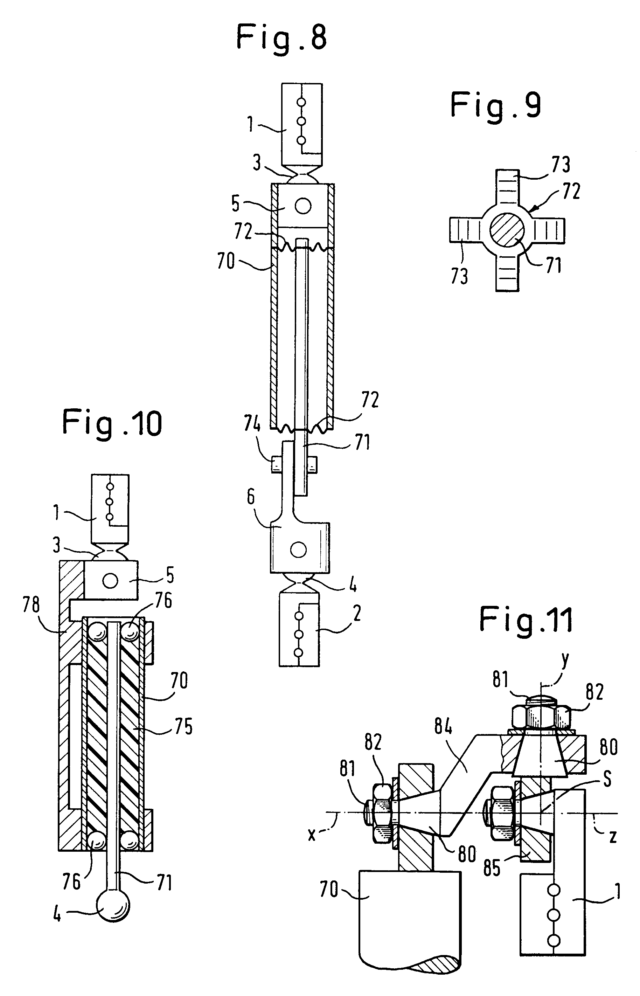

Eine ebenfalls mit Blattfedern versehene Ausführungsform der gelenkigen Verbindung ist in Figur 8 dargestellt. Dabei ist an der Gelenkhälfte 5 eine Hülse 70 befestigt und ist an der anderen Gelenkhälfte 6 eine Stange 71 befestigt, die sich konzentrisch in die Hülse 70 erstreckt. Die Hülse 70 ist mit der Stange 71 über zwei axial beabstandete Blattfedern 72 verbunden. Eine Draufsicht der Blattfeder 72 ist in Figur 9 dargestellt. Die Arme 73 der Blattfeder können gewellt bzw. gekröpft sein, um einen axialen Federweg von einigen Millimetern zu ermöglichen. Andererseits ist durch die Federn 72 ein radiales Auswandern der Stange 71 vermieden.

Die Kreuzfeder 72 kann auch durch eine Blattfeder ersetzt werden. Die Längeneinstellung zwischen der Stange 71 und der Gelenkhälfte 6 erfolgt durch eine Schraubverbindung 75.

In Figur 10 ist eine an der Kugel 4 der nicht dargestellten Gelenkhälfte befestigte Stange 71 dargestellt, die sich konzentrisch in einer Hülse 70 erstreckt. Der Ringraum zwischen der Stange 71 und der Hülse 70 ist mit einem elastischen und beidseits haftenden Werkstoff ausgefüllt, beispielsweise einer Siliconmasse. Es ist somit eine Längsverschiebung zwischen Stange und Hülse möglich und ein radiales Auswandern der Stange gegenüber der Hülse im wesentlichen vermieden. Mittels an beiden Enden der Hülse in dem Ringraum angeordneter Kugeln 76 läßt sich die Stange 71 innerhalb der Hülse 70 einwandfrei führen. Die Kugeln 76 können unmittelbar in den elastischen Kunststoff eingebettet sein.

In dieser Ausführung erfolgt die Längeneinstellung der Vorrichtung durch einen Ringkörper 78, der auf der Hülse 70 verschiebbar und einstellbar gelagert ist und der mit der Gelenkhälfte 5 verbunden ist.

In Figur 11 ist ein Mehrfach-Drehgelenk dargestellt, welches anstelle der Kugelgelenke 3, 5 bzw. 4, 6 vorgesehen ist. Die Drehgelenke bestehen aus konischen Zapfen 80 und Schraubgewinden 81. Das Lösen und Feststellen erfolgt über je eine Mutter 82. Die Verbindung der Hülse 70 mit der Klemmbacke 1 erfolgt über zwei Zwischenstücke 84 und 85, die in der dargestellten Weise mit den Drehgelenken zusammenwirken, so daß sich die einzelnen Drehachsen x, y und z der drei Gelenke in einem Punkt S schneiden. Es ergibt sich somit eine dreidimensionale Einstellbarkeit wie bei einem Kugelgelenk. Es ist jedoch um jede Achse eine gesonderte Ein- bzw. Feststellbarkeit gegeben.

Außerdem ist in Figur 11 dargestellt, daß die Klemmbacke 1 zum Befestigen an dem einen Knochenfragment parallel zur Hülse 70 liegt. Dadurch wird die axiale Baulänge der Vorrichtung stark verkürzt.

## Patentansprüche

1. Vorrichtung zum externen Festlegen von Knochenfragmenten mit zwei miteinander verbundenen Körpern (9, 10), die jeweils über ein Gelenk (3, 4; 5, 6) am Knochenfragment befestigt sind, und die gegen gegenseitiges Verdrehen gesichert und in Längsrichtung verstellbar sind, dadurch **gekennzeichnet,** daß die beiden Körper (9, 10) über ein Parallelogrammgelenk (11, 12, 13, 20, 21) miteinander verbunden sind.

2. Vorrichtung nach Anspruch 1, dadurch **gekennzeichnet,** daß die mit dem Gelenk auszuführende Längsverschiebung der Körper (9, 10) durch einen Anschlag auf kleine Werte begrenzt ist.

3. Vorrichtung nach Anspruch 1 oder 2, dadurch **gekennzeichnet,** daß jeder Körper (9, 10) einen rechteckförmigen, sich in Längsrichtung erstreckenden Steg (12, 13) aufweist und beide Stege über Laschen (20) gelenkig verbunden sind.

4. Vorrichtung nach Anspruch 3, dadurch **gekennzeichnet,** daß einer der beiden Stege (13) an einer plattenförmigen Verlängerung (11) des einen Körpers (9) längseinstellbar befestigt ist.

5. Vorrichtung nach einem der Ansprüche 1 bis 4, dadurch **gekennzeichnet,** daß die Stege (12, 13) mit Bohrungen (14, 17) versehen sind, die in Längsrichtung beabstandet und von der Längsachse in gleichen Abständen angeordnet sind und in denen Verbindungszapfen (21) drehbar gelagert sind, welche zu beiden Seiten des Gelenks an Laschen (20) befestigt sind.

6. Vorrichtung nach Anspruch 3, dadurch **gekennzeichnet,** daß ein Anschlag zwischen einem der Körper (10) und der Stirnkante eines Steges (13) vorgesehen ist.

7. Vorrichtung nach einem der Ansprüche 1 bis 6, dadurch **gekennzeichnet,** daß an dem Parallelogrammgelenk (11, 12, 13, 20, 21) eine Verstelleinrichtung angeordnet ist, mit der auf die Knochenfragmente eine Druckkraft zum Auseinanderschieben und eine Zugkraft zum Zusammenziehen aufbringbar ist.

8. Vorrichtung nach Anspruch 7, dadurch **gekennzeichnet,** daß die Verstelleinrichtung eine Klammer (30) ist, die auf das Parallelogrammgelenk aufsetzbar ist und als zweiarmiger Hebel ausgebildet ist, wobei durch Verstellen des einen Hebelarms das Parallelogrammgelenk über den anderen Hebelarm verschiebbar ist.

9. Vorrichtung nach Anspruch 8, dadurch **gekennzeichnet,** daß die Verstelleinrichtung sich quer zur Verschiebungsrichtung des Parallelogrammgelenks erstreckende, mit jeweils einem Laschenpaar (20) zusammenwirkende Arme (33) aufweist, deren Enden (34) das Laschenpaar erfassen und daß ein die Arme verbindender Steg (31) über eine Druckschraube (38) auf einem der Stege (12, 13) abstützbar ist, und daß die Verstelleinrichtung am Parallelogrammgelenk umsetzbar ist, wobei sie für das Ausüben von Druck bzw. Zug mit je einem der Laschenpaare (20) zusammenwirkt.

10. Vorrichtung nach Anspruch 9, dadurch **gekennzeichnet,** daß die von der Druckschraube (38) erzeugte Druck- bzw. Zugkraft des Parallelogrammgelenks angezeigt wird.

11. Vorrichtung nach Anspruch 10, dadurch **gekennzeichnet,** daß die Druckschraube aus einer in ein Gewinde der Klammer (30) einschraubbaren Buchse (42) und einem in der Buchse verschiebbar geführten Stift (43) besteht, der über eine Tellerfeder (46) an der Buchse abgestützt ist.

12. Vorrichtung nach Anspruch 11, dadurch **gekennzeichnet,** daß die Verschiebung des Stiftes (43) gegenüber der Buchse (42) über eine Markierung (48, 50) anzeigbar ist.

13. Vorrichtung nach einem der Ansprüche 7 bis 12, dadurch **gekennzeichnet,** daß die Klammer (30) eine Vorrichtung zum Erzeugen eines pulsierenden Druckes aufweist.

14. Vorrichtung nach einem der Ansprüche 1 bis 13, dadurch **gekennzeichnet,** daß das Parallelogrammgelenk von mindestens einer Feder in einer vorbestimmten Lage gehalten ist.

15. Vorrichtung nach Anspruch 14, dadurch **gekennzeichnet,** daß wenigstens ein Laschenpaar des Parallelogrammgelenks aus Blattfedern (60) besteht.

16. Vorrichtung nach Anspruch 14, dadurch **gekennzeichnet,** daß neben den Laschen (20) des Parallelogrammgelenks eine auf beide Körper wirkende Blattfeder (62) vorgesehen ist.

17. Vorrichtung zum externen Festlegen von Knochenfragmenten mit zwei miteinander verbundenen Körpern, die jeweils über ein Gelenk am Knochenfragment befestigt sind, gegen gegenseitiges Verdrehen gesichert und in Längsrichtung einstellbar sind, dadurch gekennzeichnet, daß zur Verbindung der beiden Körper eine sich in eine Hülse (70) erstreckende Stange (71) vorgesehen ist und die Stange mit der Hülse in Längsrichtung federnd und in Querrichtung im wesentlichen starr verbunden ist.

18. Vorrichtung nach Anspruch 17, dadurch gekennzeichnet, daß die Stange (71) mit der Hülse (70) über Blattfedern (72) verbunden ist.

19. Vorrichtung nach Anspruch 17, dadurch gekennzeichnet, daß der Ringraum zwischen der Stange (71) und der Hülse (70) mit einem elastischen und haftenden Werkstoff ausgefüllt ist.

20. Vorrichtung nach Anspruch 19, dadurch **gekennzeichnet,** daß zwischen Stange und Hülse Wälzkörper (76) angeordnet sind.

21. Vorrichtung nach einem der Ansprüche 17 bis 20, dadurch **gekennzeichnet,** daß die Längenverstellung der Vorrichtung mindestens einen die Hülse (70) umgreifenden Ringkörper (78) aufweist.

22. Vorrichtung nach einem der Ansprüche 1 bis 21, dadurch **gekennzeichnet,** daß das Kugelgelenk zur Verbindung der Körper (9, 10) mit den Knochenfragmenten aus einer Gelenkhälfte (5, 6) mit einer Pfanne zur Aufnahme der Kugel (57) der Gegengelenkhälfte versehen ist, und die Kugel mittels einer Überwurfmutter (55) festspannbar ist und das Gewinde (54) auf der Gelenkhälfte (5, 6) in zwei Halbschalen ausgebildet ist.

23. Vorrichtung nach einem der Ansprüche 1 bis 21, dadurch **gekennzeichnet,** daß das Gelenk zur Verbindung der Körper (9, 10) mit den Knochenfragmenten mit je drei Drehgelenken versehen ist.

24. Vorrichtung nach Anspruch 23, dadurch **gekennzeichnet,** daß die Drehachsen der Drehgelenke einen gemeinsamen Schnittpunkt (S) aufweisen.

25. Vorrichtung nach Anspruch 23 oder 24, dadurch **gekennzeichnet,** daß die Drehgelenke als Gelenke mit konischen Zapfen ausgebildet sind.

26. Vorrichtung nach einem der Ansprüche 22 bis 25, dadurch **gekennzeichnet,** daß die Klemmplatten zum Befestigen am Knochenfragment parallel und neben den verstellbaren Körpern (11, 12, 13; 70, 71) angeordnet sind.

## Claims

1. A device for externally adjusting bone fragments comprising a pair of members (9,10) which are secured to each other and which are anchored through a joint means (3,4; 5,6) each to a bone fragment, and which members are secured against relative rotation and are adjustable in the longitudinal direction,
characterized in that both members (9,10) are connected to each other through a parallelogram hinge (11,12,13,20,21).

2. The device of claim 1,
characterized in that the longitudinal displacement of the members (9,10) which is performed by the hinge is limited to small values by an abutment.

3. The device of claim 1 or 2,
characterized in that each member (9,10) comprises a rectangular, longitudinally extending plate (12,13), which plates are pivotally connected through links (20).

4. The device of claim 3,
characterized in that one of said plates (13) is mounted to be adjustable in length to a plate-shaped extension (11) of one of said members (9).

5. The device of one of claims 1 to 4,
characterized in that the plates (12,13) are provided with bores (14,17), which are spaced with respect to the longitudinal axis, in which bores connecting pins (21) are rotatably supported which are connected to said links (20) at either side of the hinge.

6. The device of claim 3,
characterized in that an abutment is provided between one of said members (10) and the edge of a plate (13).

7. The device of one of claims 1 to 6,
characterized in that the parallelogram hinge (11,12,13,20,21) comprises an adjusting means which is operated to either apply a compression force to the bone fragments for moving them apart or to apply a tension force for drawing the bone fragments together.

8. The device of claim 7,
characterized in that the adjusting means is a bracket (30) which is placed to the parallelogram hinge, which bracket is formed as a double-armed lever, wherein by displacing one of the lever arms the parallelogram hinge is displaced through the other lever arm.

9. The device of claim 1,
characterized in that the adjusting means comprises a pair of arms (33) extending normal to the displacement direction of the parallelogram hinge and cooperating each with a pair of links (20), the ends (34) of said arms (33) engaging said links, and that a web (31) connecting said arms is supported through a compression screw (38) on one of said plates (12, 13) and that the adjusting means can be inverted with respect to the parallelogram hinge, wherein it cooperates with one of said pair of links (20) each to excert compression or tension force.

10. The device of claim 9,
characterized in that the compression or tension force of the parallelogram hinge created by said compression screw (38) is displayed.

11. The device of claim 10,
characterized in that the compression screw comprises a sleeve (42) to be screwed in a thread of said bracket (30), and a pin (43) slidably guided in said sleeve, which pin is supported on said sleeve by a Belleville type spring (46).

12. The device of claim 11,
characterized in that the displacement of the pin (43) with respect to the sleeve (42) is displayed on marking means (48,50).

13. The device of one of claims 7 to 12,
characterized in that the bracket (20) comprises a means for generating a pulsating pressure.

14. The device of one of claims 1 to 13,
characterized in that the parallelogram hinge is held in a predetermined position by at least one spring.

15. The device of claim 14,
characterized in that at least a pair of links of the parallelogram hinge consists of blade springs (60).

16. The device of claim 14,
characterized in that in addition to the links (20) of the parallelogram hinge a blade spring (62) acting on both members is provided.

17. A device for externally adjusting bone fragments, comprising a pair of members (9,10) which are secured to each other and which members are anchored through a joint means, each to a bone fragment and which members are secured against relative rotation and are adjustable in the longitudinal direction,
characterized in that a rod (71) extending in a sleeve (70) is provided to connect both said members, and that the rod is connected to said sleeve resiliently in the longitudinal direction and substantially rigid in the transverse direction.

18. The device of claim 17,
characterized in that the rod (71) is connected to the sleeve (70) through blade springs (72).

19. The device of claim 17,
characterized in that the annular space between the rod (71) and the sleeve (70) is filled out with an elastic and adhering material.

20. The device of claim 19,
characterized in that ball members (76) are provided between the rod and the sleeve.

21. The device of one of claims 17 to 20,
characterized in that the longitudinal displacement of the device comprises at least an annular member (78) peripherally engaging the sleeve (70).

22. The device of one of claims 1 to 21,
characterized in that the ball joint for connecting the members (9,10) to the bone fragments comprises an element (5,6) having a seat for receiving a ball (57) of the counter element, and that the ball is tightened by means of a coupling nut (55) and that the thread (54) provided on the elements (5,6) is formed on a pair of semi-shells.

23. The device of one of claims 1 to 21,
characterized in that the joint means for connecting the members (9,10) to the bone fragments is provided with three pivoting joints each.

24. The device of claim 23,
characterized in that the rotational axes of the joint means have a common intersecting point (S).

25. The device of claim 23 or 24,
characterized in that the joint means are formed as joints having tapered pins.

26. The device of one of claims 22 to 25,
characterized in that the clamping plates for anchoring the bone fragments are located parallel and adjacent the adjustable members (11,12,13;70,71).

## Revendications

1. Dispositif de fixation externe de fragments d'os, comportant deux éléments (9, 10) fixés l'un et l'autre sur le fragment d'os par l'intermédiaire d'une articulation (3, 4; 5, 6), bloqués en rotation dans les deux sens et pouvant se déplacer dans le sens longitudinal, caractérisé en ce que les deux éléments (9, 10) sont reliés entre eux par l'intermédiaire d'une articulation à parallélogramme (11, 12, 13, 20, 21).

2. Dispositif suivant la revendication 1, caractérisé en ce que le déplacement longitudinal des éléments (9, 10) pouvant être exécuté avec l'articulation est limité à une faible valeur.

3. Dispositif suivant la revendication 1 ou la revendication 2, caractérisé en ce que chaque élément (9, 10) présente une entretoise rectangulaire (12, 13) orientée dans le sens longitudinal et en ce que les deux entretoises sont reliées de façon articulée au moyen d'éclisses (20).

4. Dispositif suivant la revendication 3, caractérisé en ce que l'une des deux entretoises (13) est fixée sur un prolongement (11) en forme de plaque de l'un des éléments (9), de façon à pouvoir être réglée longitudinalement.

5. Dispositif suivant l'une quelconque des revendications 1 à 4, caractérisé en ce que les entretoises (12, 13) présentent des alésages (14, 17) écartés, dans le sens longitudinal, et situés à la même distance de l'axe longitudinal, et que des axes de liaison (21) sont montés tournants dans ces alésages et sont fixés, des deux côtés de l'articulation, sur des éclisses (20).

6. Dispositif suivant la revendication 3, caractérisé en ce qu'une butée est prévue entre l'un des éléments (10) et le bord frontal d'une entretoise (13).

7. Dispositif suivant l'une quelconque des revendications 1 à 6, caractérisé en ce que, sur l'articulation à parallélogramme (11, 12, 13, 20, 21), est disposé un dispositif de réglage avec lequel on peut appliquer une force de pression sur les fragments d'os pour les écarter, et une force de traction pour les rapprocher.

8. Dispositif suivant la revendication 7, caractérisé en ce que le dispositif de réglage est un crochet (30), qui peut être monté sur l'articulation à parallélogramme et qui est réalisé sous la forme d'un levier à deux bras, le déplacement d'un des bras de levier, permettant de régler l'articulation à parallélogramme au moyen de l'autre bras de levier.

9. Dispositif suivant la revendication 8, caractérisé en ce que le dispositif de réglage présente des bras (33) disposés perpendiculairement à la direction de coulissement de l'articulation à parallélogramme, coopérant, chacun en ce qui le concerne, avec une éclisse d'une paire d'éclisses (20), et dont les extrémités (34) font prise sur la paire d'éclisses (20), en ce qu'une partie de liaison (31) reliant les bras peut s'appuyer, par l'intermédiaire d'une vis de pression (38), sur l'une des entretoises (12, 13), et en ce que le dispositif de réglage peut être déplacé contre l'articulation à parallélogramme (11), ce dispositif coopérant pour exercer la pression ou la traction, avec chaque éclisse correspondante de la paire d'éclisses (20).

10. Dispositif suivant la revendication 9, caractérisé en ce que la force de pression ou de traction de l'articulation à parallélogramme (11), exercée au moyen de la vis de pression (38), est visualisée.

11. Dispositif suivant la revendication 10, caractérisé en ce que la vis de pression est constituée par une douille (42) vissée dans un filetage du crochet (30), et par une tige (43), guidée et pouvant coulisser dans la douille, et s'appuyant sur la douille par l'intermédiaire d'une rondelle-élastique (46).

12. Dispositif suivant la revendication 11, caractérisé en ce que le coulissement de la broche (43) par rapport à la douille (42) peut être visualisé au moyen d'un repère (48, 50).

13. Dispositif suivant l'une quelconque des revendications 7 à 12, caractérisé en ce que le crochet (30) présente un dispositif réalisant une pression par impulsions.

14. Dispositif suivant l'une quelconque des revendications 1 à 13, caractérisé en ce que l'articulation à parallélogramme est maintenue dans une position prédéterminée, par au moins un ressort, .

15. Dispositif suivant la revendication 14, caractérisé en ce qu'au moins une paire d'éclisses (20) de l'articulation à parallélogramme est constituée de ressorts formés d'une lame (60).

16. Dispositif suivant la revendication 14, caractérisé en ce qu'à côté des éclisses (20) de l'articulation à parallélogramme (11), est prévu un ressort formé d'une lame (62) agissant sur les deux éléments.

17. Dispositif de fixation externe de fragments d'os, comportant deux éléments fixés l'un et l'autre sur le fragment d'os par l'intermédiaire d'une articulation (3, 4; 5, 6), bloqués en rotation dans les deux sens et pouvant se déplacer dans le sens longitudinal, caractérisé en ce que, pour la liaison des deux éléments, il est prévu une tige (71) pénétrant dans une douille (70), et en ce que la tige est reliée avec la douille de façon élastique dans le sens longitudinal et de façon essentiellement rigide dans le sens transversal.

18. Dispositif suivant la revendication 17, caractérisé en ce que la tige (71) est reliée avec la douille (70) au moyen de ressorts formés d'une lame (72).

19. Dispositif suivant la revendication 17, caractérisé en ce que l'espace annulaire entre la tige (71) et la douille (70) est rempli avec un produit élastique et adhésif.

20. Dispositif suivant la revendication 19, caractérisé en ce que des éléments de roulement (76) sont disposés entre la tige et la douille.

21. Dispositif suivant l'une quelconque des revendications 17 à 20, caractérisé en ce que le moyen de réglage longitudinal du dispositif présente au moins un élément formant bague (78) enveloppant la douille (70).

22. Dispositif suivant l'une quelconque des revendications 1 à 21, caractérisé en ce que l'articulation à rotule pour la liaison des éléments (9, 10) avec les fragments d'os est constituée d'une demi-articulation (5, 6) comportent une cuvette destinée à recevoir la rotule (57) de la demi-articulation complémentaire, en ce que la rotule peut être bloquée au moyen d'un écrou formant collerette (55) et en ce que le filetage (54) sur les demi-articulations (5, 6) est réalisé en deux demi-coquilles.

23. Dispositif suivant l'une quelconque des revendications 1 à 21, caractérisé en ce que chacune des articulations reliant les deux éléments (9, 10) avec les fragments d'os est munie de trois articulations tournantes.

24. Dispositif suivant la revendication 23, caractérisé en ce que les axes de rotation des articulations tournantes se recoupent en un point commun (S).

25. Dispositif suivant la revendication 23 ou la revendication 24, caractérisé en ce que les articulations tournantes sont réalisées sous la forme d'articulations comportant des axes coniques.

26. Dispositif suivant l'une quelconque des revendications 22 à 25, caractérisé en ce que les plaques de pincement à fixer sur les fragments d'os sont disposées parallèlement aux éléments réglables (11, 12, 13; 70, 71) et à côté d'eux.
